# EUROPEAN PATENT SPECIFICATION

(11) **EP 3 826 532 B1**
(45) Date of publication and mention of the grant of the patent: **18.09.2024**
(21) Application number: 19842188.5
(22) Date of filing: 22.07.2019
(51) Int. Cl.: A61B 5/053, A61B 5/00, A61B 5/07, A61B 5/145, A61B 17/068, A61B 17/072, A61B 17/11, A61B 17/00

(54) **DEVICE, SYSTEM AND METHOD FOR MONITORING A SITE OF INTEREST INTERNAL TO A PATIENT BODY**
VORRICHTUNG, SYSTEM UND VERFAHREN ZUR ÜBERWACHUNG EINER INTERESSIERENDEN STELLE IN EINEM PATIENTENKÖRPER
DISPOSITIF, SYSTÈME ET PROCÉDÉ DE SURVEILLANCE D'UN SITE D'INTÉRÊT INTERNE À UN CORPS DE PATIENT

(30) Priority: 23.07.2018 US 201862702007 P; 22.10.2018 WO PCT/IB2018/058214
(43) Date of publication of application: 02.06.2021
(73) Proprietor: Exero Medical Ltd, 6037607 Or Yehuda (IL)
(72) Inventor: SHOR, Erez, 5560208 Kiryat Ono (IL); POLICKER, Shai, Tenafly, New Jersey 07670 (US); BEN DAVID, Matan, 6347423 Tel Aviv (IL); KRAITZER, Amir, 4645516 Herzeliya (IL); YERED, Tal, 3704797 Pardes Hana Karkur (IL)
(74) Representative: Kasche & Partner
(86) International application number: PCT/IB2019/056247
(87) International publication number: WO 2020/021433

(56) References cited:
- WO-A1-2017/009849
- WO-A1-2017/009849
- WO-A2-2012/145548
- DE-A1- 102013 200 806
- US-A1- 2005 240 093
- US-A1- 2013 150 685
- US-A1- 2013 150 685
- PANAGIOTIS KASSANOS ET AL: "A tetrapolar bio-impedance sensing system for gastrointestinal tract monitoring", 2015 IEEE 12TH INTERNATIONAL CONFERENCE ON WEARABLE AND IMPLANTABLE BODY SENSOR NETWORKS (BSN), 12 June 2015 (2015-06-12), pages 1 - 6, XP055631088, ISBN: 978-1-4673-7201-5, DOI: 10.1109/BSN.2015.7299403

## Description

### BACKGROUND

Monitoring physiological processes following surgery is critical to ensure complications are addressed as soon as possible for proper recovery, healing and/or to provide guidance for personalized treatment. Anastomotic leak, for example, is a common, life threatening complication in gastrointestinal (GI) surgeries. Early detection is essential for proper treatment and complete recovery. Current methods of detecting anastomotic leak are either delayed, inaccurate or invasive and may compound patient trauma. Therefore, there is a need for providing efficient alternative systems and methods providing early anastomotic leak detection.

US 2013/150685 (Toth) discloses: "A system and method for monitoring a surgical site within a body, including an elongate sensory subsystem adapted to be placed or implanted near to the surgical site including a sensor for collecting physiological data from the surgical site and a communication subsystem in communication with the elongate sensory subsystem. An elongate probe extending from the communication subsystem to the vicinity of the surgical site adapted to collect physiological data pertaining to the surgical site. A method for monitoring the surgical site to determine the early onset of complications is disclosed. A method for monitoring the integrity of an organ and in particular monitoring the integrity of an anastomosis is also disclosed." (Abstract).

WO 2017/009849 (Mor Research Applications Ltd.) discloses: "Embodiments relate to an implantable device for detecting leakage of matter from a mammalian lumen, the device comprising a mesh structure that is attachable to a lumen of a mammalian. The mesh structure comprises a material or a material composition that is electrically conductive and which is measurably responsive in terms of its electrical conductivity when being subjected to leakage of matter from the lumen."

### (Abstract)

P. Kassanos, H. M. D. Ip and G. -Z. Yang, "A tetrapolar bio-impedance sensing system for gastrointestinal tract monitoring," 2015 IEEE 12th International Conference on Wearable and Implantable Body Sensor Networks (BSN), Cambridge, MA, USA, 2015, pp. 1-6, doi: 10.1109/BSN.2015.7299403, discloses: "Surgical Site Infection (SSI) imposes a significant burden clinically and compromises patient recovery. Anastomosis in the gastrointestinal (GI) tract is a particularly challenging case where failure of the anastomosis can lead to leakage, resulting in an increase in mortality rates. However early diagnosis and intervention are hampered by a lack of continuous sensing and long diagnostic intervals of current clinical practices. Tissue ischemia in the vicinity of the anastomosis has been found to be an early surrogate marker for anastomotic leakage. Electrical bio-impedance is a promising non-invasive technique for identifying and monitoring tissue ischemia. In this paper the modelling, design and validation of a bio-impedance system including compact instrumentation and a novel bio-impedance sensor optimized for mucosal tissue measurements in the GI tract are presented. The preliminary system, including the impedance probe, is validated experimentally for GI implant applications to provide early detection of tissue ischemia following GI surgery." (Abstract)

WO 2012/145548 (Hoffman) discloses: "An electrolytic biosensor for use in intracorporeal leak detection may be incorporated into a surgical drain that is installed post-operatively at the site of a wound. The electrolytic biosensor may be used to monitor drain effluent flowing through the surgical drain by measuring a fluid conductivity of the drain effluent. Certain changes, patterns, or responses of the so measured fluid conductivity values may be indicative of a physiological condition of the wound. For example, when the wound is a gastrointestinal anastomosis, the fluid conductivity values measured with the electrolytic biosensor may be used to characterize a fluid integrity of the gastrointestinal anastomosis. An electrolytic biosensor system may include an application that records conductivity data and provides notifications, such as a patient alarm, when a leak is detected." (Abstract)

### BRIEF DESCRIPTION OF THE DRAWINGS

The figures illustrate generally, by way of example, but not by way of limitation, various embodiments discussed in the present document.

For simplicity and clarity of illustration, elements shown in the figures have not necessarily been drawn to scale. For example, the dimensions of some of the elements may be exaggerated relative to other elements for clarity of presentation. Furthermore, reference numerals may be repeated among the figures to indicate corresponding or analogous elements. References to previously presented elements are implied without necessarily further citing the drawing or description in which they appear. The number of elements shown in the Figures should by no means be construed as limiting and is for illustrative purposes only. The figures are listed below.
**FIG. 1** is a schematic block-diagram depiction of a physiological phenomenon detection sequence employable by the monitoring system, according to an embodiment;
**FIGs. 2A** and **2B** depict a monitoring system coupled to a patient, according to various embodiments;
**FIG. 3** is a schematic block-diagram depiction of a physiological phenomenon detection sequence employable by the monitoring system, according to an alternative embodiment;
**FIGs. 4A** to **4C** are schematic depictions of various deployment options for deploying a sensing (also: monitoring) electrode into a patient body, according to embodiments;
**FIGs. 5A-B** and **5C-D** are schematic depictions of different stages for operably deploying and fastening a monitoring electrode of the monitoring system onto patient tissue, according to an embodiment;
**FIG. 6** is a flowchart of a method for monitoring a patient site of interest, according to some embodiments;
**FIG. 7** shows an impedance plot of a threshold-based leak detection method;
**FIG. 8** shows a plot of impedance measurement in induced leak;
**FIG. 9** shows a plot of impedance measurement in sutured leak;
**FIG. 10** shows representative electrophysiological signals measured by implanted electrodes; and
**FIG. 11** shows cross correlation value (correlation coeff) of leak animal model vs. control animal model over time.

### DETAILED DESCRIPTION

The invention is defined by the appended claims. Aspects of the present disclosure pertain to monitoring system employing an electronic sensor comprising one or more monitoring and, optionally, reference electrodes.

The leakage detection system may further comprise one or more communication devices comprising a transmitter, a receiver, and/or a transceiver (for implementing wired and/or wireless communication).

The communication device can be in communication with an external monitor and the electronic sensor. Optionally, a communication device may be external to the patient body for receiving and/or transmitting signals generated within the patient body and/or for receiving and/or transmitting signals generated outside the patient body. Optionally, a communication device may be an implantable communication device for receiving from and/or transmitting signals outside the patient body while implanted in the patient body via wired and/or wireless communication links. Optionally, a plurality of implantable communication devices implanted within the patient body may communicate with each other via wired and/or wireless communications.

Wired connections may be removable from a mammalian body (also: patient body) through a port and, as such, may be made or include nonbiodegradable, partially or fully biodegradable conductive material for the implementation of wired connections.

Optionally, a part of the electronic sensor may be biodegradable and/or biocompatible, and another part of the electronic sensor may be non-biodegradable and/or non-biocompatible.

Optionally the electronic sensor and/or the communication device components which are in contact with tissue of a patient site of interest (e.g., at a monitoring and/or reference location site) may be constructed, fully or partially, of biocompatible material and may, optionally, be partially or fully biodegradable. Optionally, non-biocompatible components may be housed within a sealed casing. Optionally, the term biodegradable may encompass the meaning of the term "bioresorbable" or "biodegradable yet non-bioresorbable".

A reference electrode may be operably engagable with a site of interest (SOI) (e.g., internal organ tissue) of the patient at a location which is different from the location of the site of interest to which the tissue-of-interest monitoring electrode is operably deployed, to provide an output that can be used as reference to the output(s) provided by the tissue-of-interest monitoring electrode. For example, the sensing or monitoring electrode may operably engage a surgical site (e.g., a tissue connection site such as, for example, an anastomosis site), and the reference electrode may operably engage a patient site of interest at a location which is remote from the surgical site. For example, the reference electrode may operable engage the patient site of interest at a location at which no anastomotic leak is expected to occur. The two different patient site of interest locations may herein be referred to as "monitoring location site", (or simply: "monitoring site"), to designate, for example, a surgical location site" (or simply: "surgical site") and "reference location site" (or simply: "reference site"). The monitoring and/or reference location sites may be internal and/or external to the patient body. The monitoring and/or reference electrodes may be implantable or non-implantable monitoring and/or reference electrodes, respectively. Optionally, the reference electrode may be located external to the patient body.

The monitoring electrode may be implantable, biocompatible and, optionally, biodegradable in full or in part. In some embodiments, only the tissue-of-interest monitoring electrode is biodegradable in full or in part whereas the reference electrode may be non-biodegradable yet biocompatible. Optionally, a monitoring electrode referred to herein can be a multi-electrode or comprise an arrangement of multiple electrodes. Reading multiple signals from a plurality of electrodes enables better localization of the physiological phenomena and thus allow spatial or temporal-spatial monitoring of the signals, providing for example an indication indicates propagation velocity if the condition further develops. In some embodiments, a monitoring electrode can be referred to as an electrochemically responsive sensing or monitoring electrode.

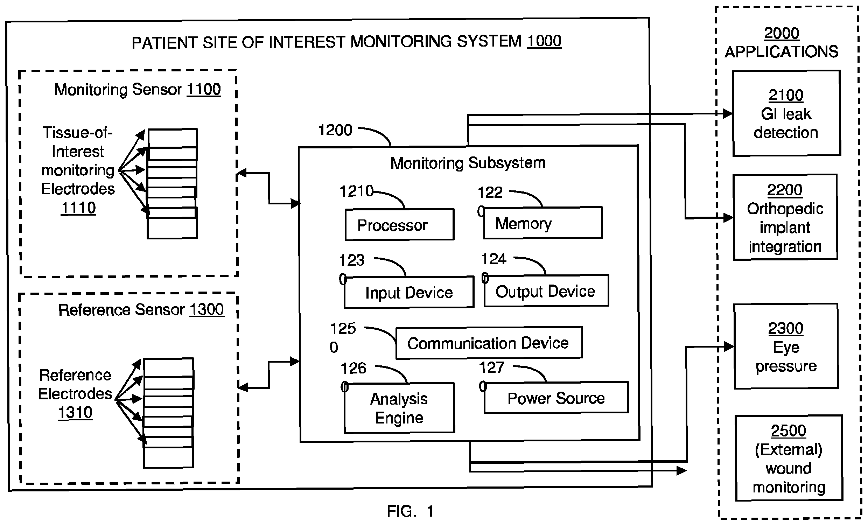

Referring now to **FIG.** 1, a patient site of interest monitoring system **1000** comprises a monitoring sensor **1100** comprising at least one monitoring electrode that is communicably coupled with a monitoring subsystem **1200.** The patient site of interest monitoring system **1000** may in some embodiments also include a reference sensor **1300** comprising at least one reference electrode **1310** that is communicably coupled with monitoring subsystem **1200.**

According to some embodiments, monitoring subsystem **1200** may include a processor **1210,** a memory **1220,** an input device **1230,** an output device **1240,** a communication device **1250,** an analysis engine **1260,** and a power module **1270** for powering the various components of patient site of interest monitoring system **1000** for the implementation of various applications **2000.**

The various components of patient site of interest monitoring system **1000** may communicate with each other over one or more communication buses (not shown) and/or wired and/or wireless communication links.

Monitoring subsystem **1200** may be operatively coupled with monitoring sensor **1100** so that changes of electrical properties of monitoring electrodes **1110** are measurable by monitoring subsystem **1200,** as outlined herein below in greater detail.

Monitoring subsystem **1200** may be operable to enable the implementation of a method, process and/or operation for allowing, for example, the detection of leakage from the lumen of an organ through an organ wall. Such method, process and/or operation may herein be implemented by an "analysis engine" of monitoring subsystem **1200,** referenced by alphanumeric label **"1260".** Analysis engine **1260** may be realized by one or more hardware, software and/or hybrid hardware/software modules, e.g., as outlined herein. A module may be a self-contained hardware and/or software component that interfaces with a larger system and may comprise a machine or machines executable instructions. For example, a module may be implemented as a controller programmed to, or a hardware circuit comprising, e.g., custom VLSI circuits or gate arrays, off-the-shelf semiconductors such as logic chips, transistors, or other discrete components, configured to cause patient site of interest monitoring system **1000** to implement the method, process and/or operation as disclosed herein. A module may also be implemented in programmable hardware devices such as field programmable gate arrays, programmable array logic, programmable logic devices or the like. For example, memory **1220,** may include instruction which, when executed e.g. by processor **1210,** may cause the execution of the method, process and/or operation for enabling, for example, the detection of leakage from a GI tract of the patient.

In some examples, analysis engine **1260** may be operable to supply artificial intelligence (e.g., machine learning functionalities) for determining if leakage occurs or not. Artificial intelligence and machine learning functionalities may be implemented, for example, by employing supervised and/or unsupervised machine learning using Support Vector Machine and/or an Artificial Neural Network. For example, a Support Vector Machine may be trained with a vector representing, for instance, 2-15 min of impedance measurement data and, optionally, with dimensionality reduced additional electrophysiological signals. For instance, the Support Vector Machine may be trained with electrophysiological signals (e.g., electrogastrography signals and/or other electro physiological signals recorded from the site of interest), for example, in the frequency domain and from which phase shifts are removed and/or which are otherwise processed to reduce dimensionality. In some embodiments, electro-physiological data representing phase, and/or phase shifts over time and/or phase differences between the signals recorded from the reference site and the signals recorded from the monitoring site may also be used as an input to analysis engine **1260.**

Power module **1270** may comprise an internal power supply (e.g., a rechargeable battery) and/ or an interface for allowing connection to an external power supply.

Input device **1230** of monitoring subsystem **1200** may be communicably coupled with monitoring electrodes **1110,** e.g., in a wired or wireless manner to allow subjecting the patient site of interest, via one or more electrode of monitoring sensor **1100,** with an input signal for stimulating the patient site of interest and, concurrently, measuring the voltage drop between at least two different locations of the same electrode(s). Monitoring subsystem **1200** may in some embodiments also be operable to sense an electrophysiological signal (e.g., electrogastrography and/or other electrophysiological signals) recorded by operably engaging electrodes with tissue of a patient site of interest. For example, specific variance characteristics (e.g., patterns) of EGG and/or other electrophysiological signals may be indicative of increased likelihood of onset of anastomotic leak and/or provide indication of an anastomotic leak occurring.

Input signals provided by input device **1230** may be direct current (DC) or alternating current (AC) signals. AC input signals may be provided at an input signal frequency ranging, for example, from 1 to 120 Hz. In some embodiments, frequency sweeping may be employed, and the output may be based on analysis of the response signal obtained over the swept input signal frequency range. Alternatively, step function input signals may also be used to obtain impedance measurement.

In some embodiments, signal processing may be employed for sensing or measuring impedance as well as, for example, for sensing a signal descriptive of electrophysiological activity of the tissue with which the electrode is operable engaged. An impedance signal may be received or sensed responsive to subjecting the electrode to an input (also: stimulation) signal. The frequency of the impedance signal corresponds with the frequency of the stimulation signal. The frequency of the stimulation signal may be selected such that the frequency of the obtained response signal (also: impedance signal) is outside the range of the frequency of the electrophysiological signal of interest. This way, the response signal and the electrophysiological signal can be separated from one another using signal filtering to allow concurrent sensing or measurement of the response signal and the electrophysiological signal using the same tissue-engaging electrode. For example, the frequency of the stimulation signal may be selected to range from 20 to 40 Hz and include, for example, 31 Hz resulting in response signals having frequencies in the range of 20 to 40 Hz, while the frequency of the electrophysiological signal may range from 0 to 12 Hz. Analysis engine **1260** may separate the signal components using signal filtering to allow for separate interpretation of the superpositioned response and electrophysiological signal components. For example, a notch filter may filter out the frequency of 31 Hz of impedance signals for allowing interpretation thereof, and a low-pass filter may be employed for filtering out a frequency range of 0-12 Hz for allowing interpretation of electrophysiological signals descriptive of, for example, GI activity.

In some embodiments, separate electrodes may be employed for measuring different types of signals such as impedance signals and electrophysiological signals

Electrical characteristics of input signals are controlled by analysis engine **1260** so that the magnitudes of electrical energy in the mammalian body are within physiologically tolerable values. A physiologically tolerable value may be, for example, an alternating current of 5 nA to 800 µA at a frequency range of 5-120 Hz.

Generally, impedance measurements may be sufficient to reliably estimate tissue condition and status. Electro-physiological signals may also be sufficient to monitor certain physiological conditions. The system may record impedance and electrophysiological information simultaneously, allowing flexibility for analysis engine **1260** which can be implemented using only a sub set of the signals (e.g., to allow comparatively lower computational complexity and smaller training datasets), or using all recorded data for enabling, for example, better sensitivity and faster detection.

Monitoring sensor **1100** may be operably coupled with the patient site of interest so that a sufficiently significant change in the material properties of monitoring electrodes **1110** causes a change in an electrical property of the monitoring electrode which is measurable and analyzable by analysis engine **1260.** Information indicative of a detection in a change in the electrical property of monitoring electrodes **1110** may be conveyed to a user (not shown) via output device **1240.** In some embodiments, analysis engine **1260** may be configured to cause output device **1240** to display values (e.g., auditory and/or visually) of the electrical properties as a function of time, e.g., within a calibrated scale, and/or provide an output of the analysis performed by analysis engine **1260** (e.g., provide an audible and/or visual alert).

In some embodiments, communication device **1250** may be equipped with a transmitter (not shown) and, optionally, with a receiver and/or a transceiver, e.g., for allowing the transmission of inputs or stimulation signals to monitoring electrodes **1110** and, optionally, to reference electrodes **1310.** Analysis engine **1260** may control the generation of the input signals.

In some embodiments, communication device **1250** enables the transmission of response signals carrying data ("electric-property-data") that is descriptive of a change of the electrical properties of monitoring sensor **1100** from monitoring electrodes **1110** to analysis engine **1260.**

In some embodiments, electrophysiological (e.g., electrogastric) signals may be transmitted via communication device **1250** to analysis engine **1260** for the analysis thereby.

It is noted that although components of patient site of interest monitoring system may be illustrated as being implemented by a single component, this should by no means be construed in a limiting manner. For example, components of patient of site of interest monitoring system can be deployed to be executed on one site or distributed across multiple sites and operably interconnected. For instance, separate processors and memories may be allocated to analysis engine **1260,** and separate communication devices may be implemented for implementing communication device **1250.**

It is noted that in some embodiments, one or more components of monitoring subsystem **1200** may be internal and one or more components may be external to the mammalian body. For example, certain processors, memories, input devices, communication devices and/or our power sources may be internal to the mammalian body and certain processors, memories, input devices, communication devices and/or our power sources may be outside the mammalian body.

For example, communication device **1250** may be coupled with or include a transmitter (not shown) that may be operably positionable within mammalian body. Optionally, electric-property-data may be transmitted from within the mammalian body to the outside of mammalian body wired and/or wirelessly over communication link (not shown) for further analysis by analysis engine **1260.**

In embodiments, some of monitoring subsystem **1200** functionalities may be implemented by a multifunction mobile communication device also known as "smartphone", a personal computer, a laptop computer, a tablet computer, a server (which may relate to one or more servers or storage systems and/or services associated with a business or corporate entity, including for example, a file hosting service, cloud storage service, online file storage provider, peer-to-peer file storage or hosting service and/or a cyberlocker), personal digital assistant, a workstation, a wearable device, a handheld computer, a notebook computer, a vehicular device, a stationary device and/or a home appliances control system.

The term "processor" as used herein may additionally or alternatively refer to a controller. Such processor may relate to various types of processors and/or processor architectures including, for example, embedded processors, communication processors, graphics processing unit (GPU)-accelerated computing, soft-core processors and/or embedded processors.

According to some embodiments, memory **1220** may include one or more types of computer-readable storage media. Memory **1220** may include transactional memory and/or long-term storage memory facilities and may function as file storage, document storage, program storage, or as a working memory. The latter may for example be in the form of a static random access memory (SRAM), dynamic random access memory (DRAM), read-only memory (ROM), cache or flash memory. As working memory, memory **1220** may, for example, process temporally -based instructions. As long-term memory, memory **1220** may for example include a volatile or non-volatile computer storage medium, a hard disk drive, a solid state drive, a magnetic storage medium, a flash memory and/or other storage facility. A hardware memory facility may for example store a fixed information set (e.g., software code) including, but not limited to, a file, program, application, source code, object code, and the like.

Communication device **1250** may for example include I/O device drivers (not shown) and network interface drivers (not shown). A device driver may for example, interface with a keypad or to a USB port. A network interface driver may for example execute protocols for the Internet, or an Intranet, Wide Area Network (WAN), Local Area Network (LAN) employing, e.g., Wireless Local Area Network (WLAN)), Metropolitan Area Network (MAN), Personal Area Network (PAN), extranet, 2G, 3G, 3.5G, 4G including for example Mobile WIMAX or Long Term Evolution (LTE) advanced, and/or any other current or future communication network, standard, and/or system.

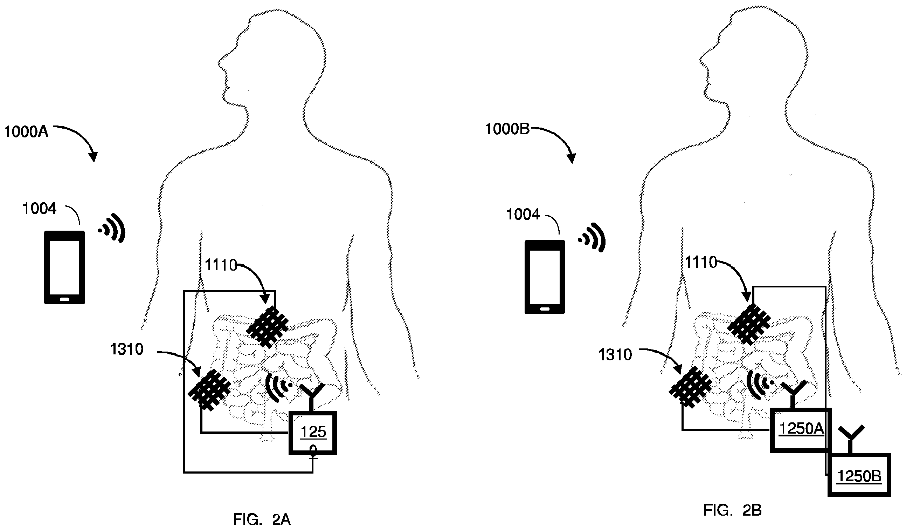

Reference is now made to **FIGs. 2A** and **2B****,** schematically illustrating various embodiments of a patient site of interest monitoring system **1000.** One embodiment of patient site of interest monitoring system **1000** is herein designated by alphanumeric reference **"1000A",** and another embodiment of monitoring system **1000** is herein designated by alphanumeric reference **"1000B".** Merely to simplify the discussion that follows, without be construed in a limiting manner, the description may refer to "monitoring system **1000".**

As shown in **FIGs. 2A** and **2B****,** patient site of interest monitoring system **1000** includes a tissue-of-interest or SOI monitoring electrode **1110** that is operably coupleable adjacent to or such to engage with a site of interest internal to an animal (e.g., human) body, herein also referred to as "patient body". Such site of interest may include, for example, an internal organ, a surgical intervention site (e.g., a tissue reconnection site such as an anastomosis site, a sleeve gastrectomy site, etc., a site which is prone to wound dehiscence or other tissue breakdown (e.g., due to tissue reconnection by employing, for example, staples, sutures, etc.), a hernia closure site, and/or any other physiological phenomenon expected or suspected to occur at the site of interest, and which physiological phenomenon may, for example, cause a measurable change in a characteristic (e.g., an electrical property) of a monitoring electrode, e.g., due to corrosion, (bio-)degradation, mechanical failure of the electrode(s) and/or the like. For example, a monitoring electrode may start bio-degrading or its biodegradation profile or its physical properties may otherwise change (e.g., accelerate or decelerate) as a result of and, e.g., in correspondence, with the onset or occurrence of a physiological phenomenon being monitored. Hence, occurrence and timing of the physiological phenomenon at the site of interest may be measurable by detecting a change in one or more characteristics of the monitoring electrode.

In some embodiments, tissue-of-interest monitoring electrode **1110** may, for example, be implantable in proximity of operably engaged with an anastomosis site of the GI track or any other surgical tissue connection site or other internal body site of interest, and may be implanted during Gastrointestinal (GI) surgery, for example. In some other embodiments, tissue-of-interest monitoring electrode **1110** may be operably integrated with a device extending from the internal body site of interest (or vicinity thereof) inside the patient's body to the outside of the patient's body, e.g., to the vicinity of the patient's skin surface. Such device may include, for example, a fluid drainage catheter that is operably positioned to drain fluids from the internal site of interest to the outside of the patient's body.

In some embodiments, a monitoring electrode **1110** and/or reference electrode **1310** may be biodegradable and/or biocompatible in full or in part. In some embodiments, a partially biodegradable monitoring electrode(s) **1110** and/or reference electrode(s) **1310** may comprise at least two parts, namely, a fully biodegradable part for engaging with tissue of a site of interest until the biodegradable part is degraded, and a partially bio-degradable or non-biodegradable part that can be retracted or removed after the monitoring period is completed. In some embodiments, a reference electrode is non-biodegradable.

In some embodiments, tissue-of-interest monitoring electrode **1110** may be biodegradable in full or in part yet not necessarily implantable (e.g., biocompatible) if it is designed to be operably positioned outside the patient's body, e.g., inside or as part of the fluid drainage catheter. For example, monitoring electrode **1110** may be operably coupled (e.g., embedded in the fluid drainage catheter such that the monitoring electrode can make contact with fluid flowing in the fluid path of the fluid drainage catheter.

A reference electrode **1310** may be employed for deployment at a different site of interest to provide an output that can be used as reference to the outputs provided by tissue-of-interest monitoring electrode **1110.** Reference electrode **1310** may also be implantable, biocompatible and optionally biodegradable in full or in part. Reference electrode **1310** may for example be employed to improve accuracy of a specificity test, provide reference for spatial or temporal-spatial propagation of monitoring electrode dynamic electrical characteristics and/or of other methods that may be employed for performing diagnostic tests. In implementations where tissue-of-interest monitoring electrode **1110** is incorporated in a fluid drainage catheter, reference electrode **1310** may be located downstream and/or upstream of tissue-of-interest monitoring electrode **1110.** The terms "upstream" and "downstream" as used herein in the context of a drainage catheter refers to a fluid drainage direction. In some embodiments, reference electrode **1310** may be deployed in a different, "reference" catheter that is operably coupled with the patient. Optionally, reference electrode **1310** may be biodegradable (fully or partially) or non-biodegradable and, in addition, not necessarily biocompatible), for example, if it is designed to be operably positioned outside the patient body, e.g., inside or as part of another fluid drainage catheter.

In some embodiments, signals received from the reference electrode may be used for performing self-calibration of components of the patient site of interesting monitoring system. For example, signals received from the reference electrode may be used for adapting at least one adverse-phenomenon output criterion based on which, for example, an output is provided for indicating that anastomotic leak occurs or not.

In some embodiments, monitor **1004** may provide artificial intelligence (including, e.g., machine learning) functionalities for adaptively changing the at least one adverse-phenomenon output criterion. The artificial intelligence functionalities may be based on patient-related characteristics.

As shown in **FIGs. 2A** and **2B****,** monitoring system **1000** may optionally include one or more communication devices **1250** for communicably linking between tissue-of-interest monitoring with a monitor **1004** and further for communicably linking reference electrodes 1**310** with monitor **1004.**

Communication device **1250** can include an active and/or a passive transmitter, receiver and/or transceiver. In some embodiments, communication device **1250** can be selectively switchable from an active to passive transmission mode and vice versa. Some or all components of communication device **1250** may be fully or partially biodegradable.

Monitor **1004** may incorporate functionalities of analysis engine **1260** and of output device **1240** which were outlined with respect to **FIG. 1****.**

In patient site of interest monitoring system **1000A,** a communication device **1250** may be communicably coupled with tissue-of-interest monitoring and reference electrodes **1110** and **1310.** In another example of monitoring system **1000B,** a first communication device **1250A** may be communicably coupled with tissue-of-interest monitoring electrodes **1110,** and a second communication device **1250B** may be communicably coupled with reference electrodes **1310.** First and second communication devices **1250A** and **1250B** may be communicably linked with each other in a wired and/or wireless communication to allow for in-body transmission of signals between the communication devices. In-body signal transmission may be employed to implement, for example, calibration, feedback, data fusion, noise-reduction, and/or other signal processing and/or analysis applications, for example, by analysis engine **1260** implemented by one or more implanted processors and/or memories.

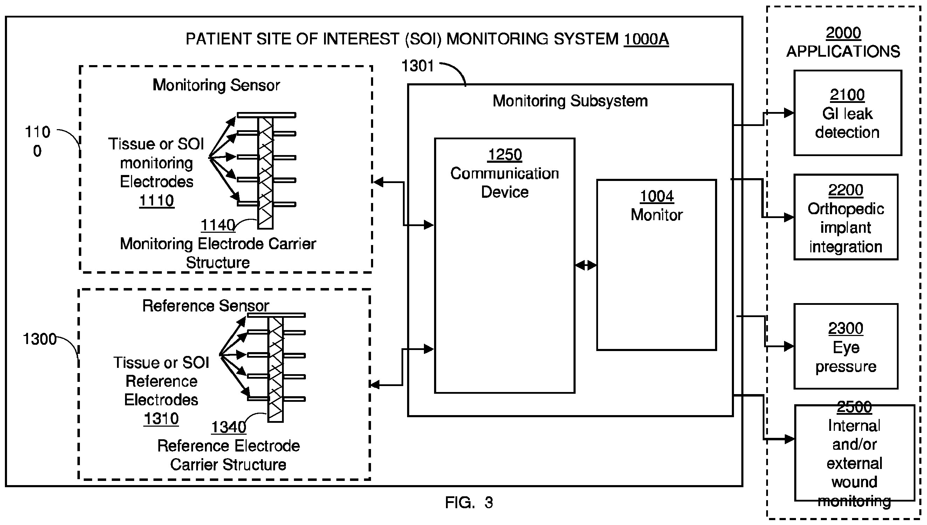

As schematically shown in **FIG.** 3 and mentioned herein with respect to **FIGs. 2A** and **2B****,** components of a monitoring subsystem **1301** may be configured to implement a communication device **1250** and a monitor **1004** to controllably subject tissue-of-interest monitoring with input signals via tissue-of-interest (TOI) monitoring and reference electrodes **1110** and **1310,** respectively, with input or stimulation signals, and is further operable to communicate response signals relating to the input signals and which are received from tissue-of-interest monitoring and reference electrodes **1110** and **1310,** respectively, to monitor **1004** which may be located outside the patient body.

For example, one or more tissue-of-interest monitoring electrodes **1110** and, optionally, one or more reference electrodes **1310,** may be in communication with communication device **1250.** Communication device **1250** may be communicably linked (wired and/or wirelessly) with monitor **1004** for providing monitor **1004** with signals generated by tissue-of-interest monitoring electrodes **1110** and, if applicable, by reference electrodes **1310.** Monitor **1004** may be operable to process signals received from tissue-of-interest monitoring electrodes **1110** and, if employed or applicable, may also be operable to process signals received from reference electrodes **1310.**

Monitor **1004** is operable to automatically or semi-automatically provide, based on the received response signals, an output pertaining to a physiological phenomenon (e.g., an or potentially adverse physiological phenomenon or condition) that may, for example, adversely affect the patient's health (e.g., wound dehiscence, anastomotic leakage, etc.) such as, for example, a warning upon detection of anastomotic leakage.

Response signals sent to monitor **1004** may be descriptive of, for example, impedance, capacitance, current flow and/or change thereof in response to detected changes of a physiological condition of the patient. In some embodiments the response signal sensed by any of the electrodes can be communicated based on the principle of electromagnetic induction. For example, patient site of interest monitoring system **1000** may comprise an implanted or implantable conductive coil (not shown). The implantable conductive coil may be part of communication device **1250,** for example. Leak detection may further comprise an external conductive coil (not shown). The external coil may be part of or otherwise be operably coupled with monitor **1004.** The internal and external coil are positioned or are operably positionable relative to each other such that a change in current in one coil is picked up by the other coil through electromagnetic induction. The internal coil may be biocompatible and can be partially or fully biodegradable. In some embodiments, the internal coil may be responsive to changes in the patient's physiological characteristics. For example, the internal coil biodegrades or biodegrades at an accelerated or slower pace when being subjected to matter or (e.g., biological) substance that is a manifestation in a physiological phenomenon occurring in the patient's body. For example, characteristics (e.g., conductive or other electric characteristics) of the internal coil may change when coming in contact with fluid leaking from the GI tract. Changes in the internal coil's electric characteristics may manifest themselves in measurable variations of the electrical output signals that are output by the external coil. The implantable internal coil may thus embody a monitoring electrode, and the internal/external coil arrangement may embody an electronic sensor for detecting GI leak, for example.

Monitor **1004,** which may be handheld, may comprise circuitry (e.g., a memory and a processor) for processing and/or analyzing response signals received from tissue-of-interest monitoring electrode **1110** and, optionally, of reference electrode **1310)** to determine, based on the received signals, an output pertaining to a physiological condition of the site of interest internal to the patient body.

For example, monitor **1004** may be operable to employ a bandpass filter on the received signals. For instance, if electrodes are subjected to a stimulation input signal at 31 Hz, response signals may be bandpass or lowpass filtered at 0.5-15 Hz. The band pass filter on the received signals recorded from the site of interest can be adaptively calibrated to consider characteristics of the signals recorded from the reference site for a computationally efficient reference-based condition detection algorithm.

For example, monitor **1004** may determine, based on the received signals, if leakage occurs or not. For instance, monitor **1004** may provide, based on the received signals, a warning if characteristics of the received signals meet at least one adverse-phenomenon output criterion such as, for example, an adverse leak warning output criterion (e.g., an output indicating that anastomotic leak occurs); an output indicative of the likelihood or probability of an adverse physiological phenomenon leak to occur within a certain time period (i.e., predicting the onset of anastomotic leak); provide an output indicative of how a current treatment condition should be altered such to reduce (e.g., minimize) the likelihood or probability of an adverse physiological phenomenon (e.g., condition or state) to occur or develop, or prevent occurrence of anastomotic leak; provide an output indicative of supplementary diagnostic test to be performed for determining if leakage occurs or not; and/or the like.

Such adverse-phenomenon output criterion may pertain to sensed variations in electrical signal characteristics and may be static, a dynamic or adaptive leak-related output criterion. A static criterion is predetermined that remains constant. A dynamic criterion is forcefully changed, for example, at a certain time of day, or a certain day of the year. An adaptive criterion is changed in response to changes in, for example, physiological characteristics of the patient body and/or the patient body's environment, and may vary depending on a variety of parameters.

For example, a leakage warning may be provided if monitor **1004** detects a voltage drop below or voltage increase above a corresponding "low or high electrical characteristic warning threshold"; and/or if monitor **1004** detects a drop below or increase above a corresponding "low or high electrical characteristic warning threshold range". The term "threshold" as used herein may refer to a predetermined threshold, a reference calibrated threshold, a moving threshold (e.g., linearly and/or non-linearly moving threshold) and/or any combination thereof. An adverse-phenomenon output criterion may in some implementations additionally or alternatively relate to a threshold relating to electrophysiological (e.g., electrogastrography) signal variance and/ or other signal characteristics. For example, lower cross-correlation between electrophysiological signals recorded from the reference site vs. cross-correlation between signals recorded in the monitoring site may be indicative of increased likelihood of onset of anastomotic leak and/or provide indication of an anastomotic leak occurring. Optionally, analysis engine **1260** takes into account food intake when taking into account electrophysiological to determine if an adverse-phenomenon output criterion is met.

For example, a leakage warning may be provided if monitor **1004** detects an impedance drop below or impedance increase above a corresponding "low or high electrical characteristic warning threshold"; and/or if monitor **1004** detects a drop below or increase above a corresponding "low or high electrical characteristic warning threshold range".

In certain embodiments, monitor **1004**and/or communication device **1250** may be operable to communicate with additional computing devices for providing expanded range of monitoring. Such computing device can include, for example, a multifunction mobile communication device also known as "smartphone", a personal computer, a laptop computer, a tablet computer, a server (which may relate to one or more servers or storage systems and/or services associated with a business or corporate entity, including for example, a file hosting service, cloud storage service, online file storage provider, peer-to-peer file storage or hosting service and/or a cyberlocker), personal digital assistant, a workstation, a wearable device, a handheld computer, a notebook computer, a vehicular device and/or a stationary device.

In some embodiments, patient site of interest monitoring system **1000** is operative at low currents which are considered or known to be safe to the patient such as, for example, below 10 mA. For example, patient site of interest monitoring system **1000** is operative at low currents below low-threshold limits in the sense that such currents will have minimal effect on nerves or muscles including, for example, electrical currents below 1 mA (e.g., for muscles), or with currents below, for example, 10 nA (e.g., for nerves). In some embodiments, narrow current pulses may be utilized known to have reduced, minimal or no effect on muscles such as pulse widths shorter than, for example, 1 ms or pulse widths which are shorter than, for example, 100 µs or other pulse widths known to have reduced, minimal or no effect on, e.g., human nerves, such as pulse widths shorter than, for example, 100 µs. Low-threshold effects may be assured by applying currents at sufficiently low pulse widths. In some embodiments, a pulse width may exceed low-thresholds pulse-width limit, if the applied current is sufficiently low. In some embodiments, the applied current may exceed sub-threshold current limits if the pulse width with the currents are applied is sufficiently narrow. In some embodiments, electric pulses applied may be applied at frequencies which are below a corresponding low-threshold frequency limit.

Additional reference is made to **FIG. 3****.** In some embodiments, one or more tissue-of-interest monitoring electrodes **1110** and reference electrodes **1310** may be operably coupled to a monitoring electrode carrier structures **1140** and a reference electrode carrier structure **1340,** respectively. The electrode carrier structures facilitate securely and operably engaging (e.g., operably non-removably or removably coupling or fastening) the tissue-of-interest monitoring and reference electrodes **1110** and **1310,** respectively, with organ tissue.

Optionally, the one or more TOI monitoring electrodes **1110** may herein be referred to in the singular as a "tissue-of-interest monitoring sensor **1100",** and the one or more TOI reference electrodes **1310** may be referred to in the singular as a "reference sensor **1300".** Optionally, the one or more tissue-of-interest monitoring electrodes **1110** and a tissue-of-interest monitoring electrode carrier structure **1140** to which the one or more tissue-of-interest monitoring electrodes **1110** are coupled, may herein be referred to as a "tissue-of-interest monitoring sensor **1110".** Analogously, the one or more reference electrodes **1310** and a reference electrode carrier structure **1340** to which the one or more reference electrodes **1310** may be coupled may herein be referred to as "reference sensor **1300".** Such (leak or reference) electrode carrier structure can be in the form of a mesh, for example. In some embodiments, tissue-of-interest monitoring electrodes **1110** may be operably coupled with monitoring electrode carrier structure **1140** to form a tissue-of-interest monitoring sensor **1100.** Analogously, reference electrodes **1310** may be operably coupled with a reference electrode carrier structure **1340** to form reference sensor **1300.**

In some embodiments, material(s) that are used for constructing a mesh may constitute a part of tissue-of-interest monitoring and/or reference electrodes **1110** and **1310.** In some embodiments, tissue-of-interest monitoring and/or reference electrodes **1110** and **1310** may be arranged to form, respectively, tissue-of-interest monitoring and reference carrier structures **1140** and **1340.**

In some embodiments, sensing elements of an (e.g., implantable) electronic sensor such as the monitoring electrode and/or a mesh may have a material thickness and/or other properties to ensure detectability of changes of a physiological phenomenon in a patient. The sensing element(s) may be implantable or non-implantable. Optionally, a part of a sensing element may be implantable, and a part may be non-implantable. Optionally, a part of the sensing element may be biodegradable, and another part may be non-biodegradable. For example, to attain a detectable corrosion rate for a desired period of time while retaining mechanical properties of the monitoring electrode, for instance, a metal alloy wire of the monitoring electrode may have a diameter ranging, for example, from, 100 µm to 800 µm. In this case, analysis engine **1260** may consider the non-linear corrosion pattern of the electrode and its effect on the sensor's impedance. It is noted that the electrode or components thereof may include a cable, a sheath metal, a film, a single wire, and/or the like. The sensing elements may include one or more electrodes, meshes, rods, strings, wires, cables, machined sheath metals, film, and/or the like..

The term "mesh" as used herein, may refer to a two- or multidimensional semipermeable structure of closely-spaced holes, which is composed of a plurality of elongated and interconnected elements, such as fibers, strands, struts, spokes, rungs made of a flexible/ductile material, which are arranged in an ordered (matrix, circular, spiral) or random fashion to form e.g., a two-dimensional sheet or a three-dimensional object. In some embodiments, the term "mesh" is intended to include an element having an openwork fabric or structure, and may include but is not limited to, an interconnected network of wire-like segments, a sheet of material having numerous apertures and/or portions of material removed, or the like. Accordingly, the term "mesh" may also refer to a matrix or a net structure. A wire-like segment may for example comprise monofilaments and/or braided fibers. In some embodiments, the mesh may comprise or embed one or more electrodes.

In some embodiments, by "closely-spaced holes" it is meant to refer to a spacing of e.g., 1 mm, 2 mm, 5 mm, 10 mm, 15, mm, 20 mm, 30 mm, 40 mm, 50 mm, 60 mm, 70 mm, 80 mm, 90 mm, 100 mm, 110 mm, 120 mm, 130 mm, 140 mm, 150 mm, 160 mm, 170 mm, 180 mm, 190 mm, or 200 mm, including any value and range therebetween.

According to some embodiments, certain meshes may be composed of fibrous elements which come in direct physical contact with each other at each intercrossing junction constituting the mesh.

In some embodiments, tissue-of-interest monitoring electrodes **1110,** reference electrodes **1310,** monitoring electrode carrier structure **1140** and/or electrode carrier structure **1340** comprise or are made of conductive, biocompatible and/or (bio-) degradable material(s). For example, the mesh or the wire structure comprises a core structure coated with conductive, biocompatible and/or biodegradable material(s). In some embodiments, the core comprises one or more metals. Optionally, the mesh structure comprises non-conductive polymer fibers that are interwoven with conductive, biocompatible and/or biodegradable material(s). Optionally, the wire (or the mesh) has a uniformly porous architecture so that degradation of the wire (or the mesh) can be progressed uniformly. Optionally, the mesh has a dimension of 0.1 mm to 500 mm x 0.1 mm to 500 mm, including any value and range therebetween.

Tissue-of-interest monitoring electrodes **1110,** reference electrodes **1310,** monitoring electrode carrier structure **1140** and reference electrode carrier structure **1340** can be made of any suitable material including, for example, non-biodegradable, partially biodegradable, or fully biodegradable material such as, for example, magnesium-based material, magnesium alloys, stainless steel, carbon tip, platinum, platinum-iridium alloy, gold, and/or any type of biodegradable and/or biocompatible alloy or metal. Tissue-of-interest monitoring electrode carrier structures **1140** and reference electrode carrier structures **1340** may for example comprise, in addition to the metal components Vicryl^{™}, Polyglycolic acid:Trimethylene carbonate (PGA:TMC), PGA, and/or non-(bio)degradable materials that are employable in implantable devices, and/or any other suitable material.

Mg impedance for example is increasing due to the generation of corrosion products (metal oxides and metal hydroxides) covering some of the effective surface area of the electrode. Due to corrosion, effective electrode surface area is changing, mainly decreasing, thus making the conductive contact area of the electrode with the tissue smaller.

Tissue-of-interest monitoring electrode carrier structure **1140** and reference electrode carrier structure **1340** may be operably fastened to organ tissue by employing, for example, various fixation elements and/or methods including, e.g., glue, adhesives and/or sutures. For example, barbed Polyglyconate biodegradable suture may be threaded into a mesh to facilitate local fastening and for stabilizing monitoring sensor **1100** and reference sensor **1300** in place of corresponding sites of interest, without puncturing or otherwise inadvertently damaging tissue structure such as the GI tract.

As noted, monitor **1004** comprises circuitry (e.g., a processor and a memory) that is configured to adaptively change at least one adverse-phenomenon output criterion. The at least one adverse-phenomenon output criterion may be changed adaptively based on, for example, patient-related characteristics deemed relevant to proper evaluation of patient recovery. Patient-related characteristics can include, for example, information about the patient's medical history (e.g., type(s) of surgical GI procedure(s) which the patient underwent); social characteristics (e.g., gender; age; profession; income), and/or the like. Patient-related characteristics may also include measured physiological characteristics such as, for example, biometric data; systolic blood pressure; diastolic blood pressure; mean arterial pressure; pulse rate; breathing rate; breathing pattern; oxygen saturation level; glucose level; electrical property of the patient's skin (e.g., conductivity, resistance); weight, body-mass index (BMI) pH level; concentration of one or more selected analytes in bodily fluid (e.g., magnesium, calcium, sodium, salts, glucose, and/or hormones); motor function; body temperature; sweat rate; electrocardiogram; myocardiogram; electroencephalography; capnography values; a cognitive ability of the patient; and/or the like. Bodily fluid can include blood, sweat, tears and/or saliva. In some embodiments, environmental parameters may be taken into account which can include, for example, location, temperature, humidity, room particle count, pressure level of the environment in which the patient is located and/or the like. In some embodiments, patient-related characteristics may pertain to third party data descriptive of information that may be deemed relevant for assessing patient recovery. Such third party data include data of other patients, data associated with relatives of the patient, and/or the like.

Although, embodiments of the present application pertain to the detection of anastomotic leak, the systems and methods disclosed herein may also be applied in the monitoring of various other health applications in which internal (e.g., adverse) physiological phenomena effecting electronic parameters or characteristics like impedance, phase shift, capacitance, and/or current flow voltage potential, can be rendered into electronic data. In particular the system and method described herein translates physiological conditions into data, allowing close monitoring of a surgical site for healing and recovery, and early detection of complications, namely inflammatory and/or infectious complications. Various example set forth in under "applications" **2000,** include, *inter alia,* lumen leak detection **2100,** orthopedic implant integration **2200,** eye pressure **2300,** and/or (e.g., head, sternum) other internal and/or external wound monitoring **2500.** Further applications **2000** may include, for example, monitoring healing; post tumor resection monitoring of organs with respect to recurrence; monitoring indications related to signs of rejection of transplanted organs to adjust medication such as immune-depressive pharmacological therapy dosing; monitoring for infections adjacent to any foreign surgical implants; monitoring high-risk surgical wounds (e.g., contaminated wound areas, groin, trauma wounds; monitoring areas of lymphatic dissections; monitoring areas of lymph node resections; monitoring urinary bladder function; monitoring stomach, small bowel and/or large bowel functions; monitoring sphincters including, for example, lower esophageal sphincter, pylorus, anal sphincter, and/or the like; monitoring vascular functions including, for example, renal artery and/or carotid tone monitoring; monitoring of the peritoneum, e.g., for detecting postoperative peritonitis; monitoring the mediastinum and/or the pleural cavity for postoperative mediastinitis and pleuritis and/or empyema; and/or the like.

It should be noted that while certain aspects of the present disclosure pertains to the detection of GI leaks, this should by no means be construed in a limiting manner. Accordingly, devices, systems and methods described herein may also be employed for detecting other physiological phenomenon occurring at or in the vicinity of a site of interest and which can have a measurable effect on electrical characteristics of monitoring electrodes. For example, system and methods disclosed herein can be employed in the monitoring of internal organs such as the pancreas, liver or bladder to detect a leak, evaluate healing and/or changes in inflammatory and/or malignant conditions. Furthermore, a monitoring electrode can be implanted in a site in which eminent physiological changes are expected or suspected in order to detect occurrence timing of said changes or to provide an indication that such changes are not occurring as expected.

In some embodiments the rate of biodegradation of a part of entirety of the system will be set in line with the expected timing of expected leak or other said expected or suspected physiological changes. Specifically, in some embodiments the implantable parts in the vicinity of the internal body site of interest in which the leak or other physiological phenomenon is expected or suspected are designed to bio-degrade at a similar timing of the expected physiological phenomenon, whereas the other implantable parts that reach away from the said site may be made to be biocompatible but not biodegradable.

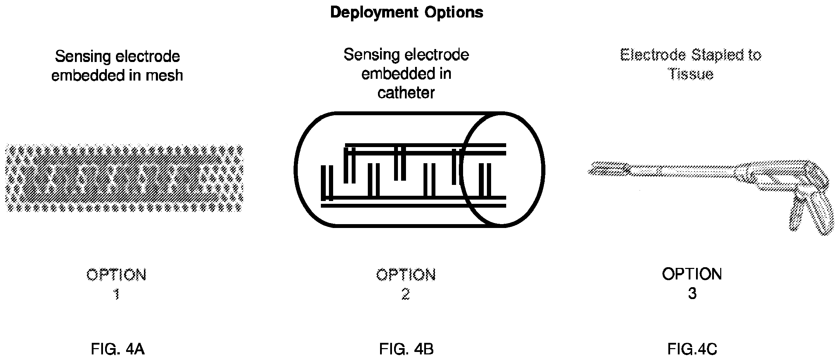

As shown in **FIG. 4A****,** a first Option **1** is directed at placing a monitoring electrode in a mesh and operably fastening them to a site of interest for the monitoring thereof.

As shown in **FIG. 4B****,** a second Option **2** is directed to embedding a sensing or monitoring electrode in a catheter, for example, a post-surgical drain catheter, to bring the sensing in contact with liquids that may be drained from the site of interest. Liquids drained from the site of interest may reflect a physiological condition such as, for example, tissue breakdown, wound dehiscence, which may manifest itself in anastomotic leak.

As shown in **FIG. 4C****,** a third Option **3** is directed to securing the monitoring electrodes to an body site of interest with staples, e.g., as is known in the art.

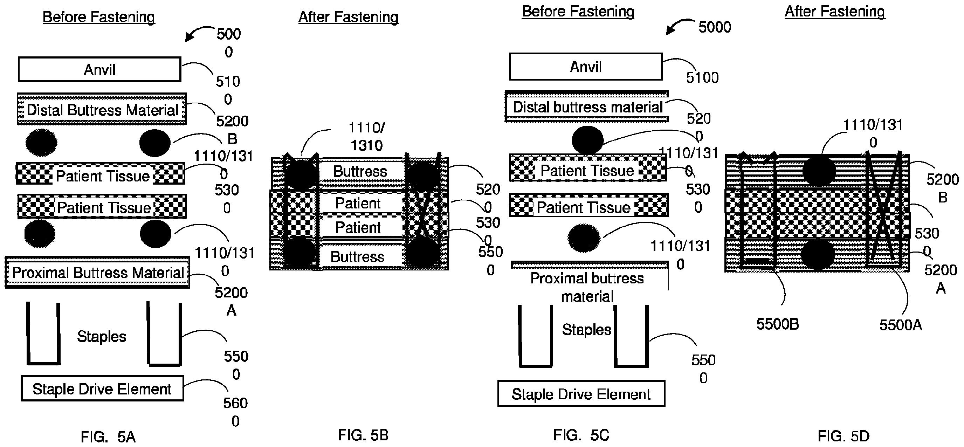

**FIGs. 5A-5B** depict different stages of stapling tissue-of-interest monitoring and/or reference electrode **1110/1310** of patient site of interest monitoring system **1000** into a deployment position in patient tissue **5300** with a surgical stapler **5000,** according to some embodiments. Staple drive element **5600** is operable to drive staples **5500** (e.g., staples **5500A** and **5500B)** against an anvil **5100** of surgical stapler **5000** subjecting, in turn, the application of a force onto staple **5500** causing the staple to contort upon contact with anvil **5100** such that the legs of the staple are bent within staple clinching pockets (not shown) of anvil **5100** relative to the staple's backspan while and/or after the legs traverse the patient's tissue to thereby secure distal buttress material **5200A** against tissue **5300** and to secure the backspan to proximal buttress material **5200B** against the patient's proximal tissue portion **5300.**

As shown schematically in **FIGs 5A-5B****,** each staple **5500** may in some embodiments be applied in alignment with the monitoring electrode(s) such that the staple directly engages with the monitoring electrode(s) for the fastening thereof with the patient's tissue. Sensing/monitoring electrode may be present at both sides of the staple line, or only one side.

As shown schematically in **FIGs. 5C-5D****,** the monitoring electrode(s) may in some embodiments be positioned between two staples **5500** such that the monitoring electrodes are secured to the patient's tissue merely by the force the staples apply for securing the proximal and distal buttress materials to the tissue. For example, the monitoring electrodes are not directly fastened onto the patient tissue but secured through the fastening of the buttress material. In some embodiments, no buttress material may be employed. It should be appreciated that in certain deployments a circular stapler may be employed. A swine model was employed in which retractrable and resorbable monitoring electrodes were implanted and engaged with a leak site at a descending colon location, and in which also retractable and resorbable reference electrodes were implanted at a reference site in the ascending/spiral colon.

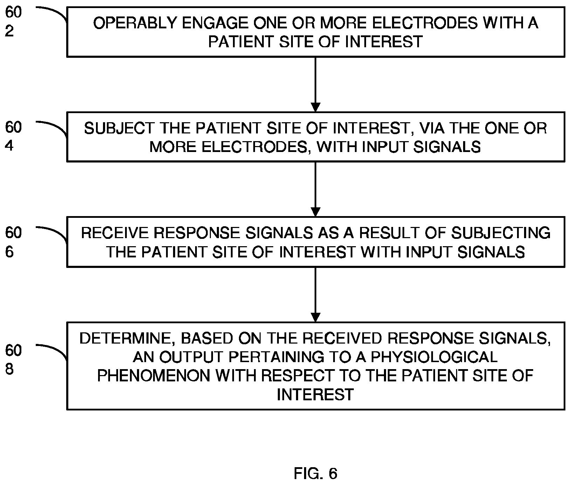

Further referring to **FIG. 6****,** a method of monitoring a patient site of interest comprises, according to some embodiments, operably engaging one or more electrodes with a patient site of interest (block **602).**

In embodiments, the method may further comprise subjecting the patient site of interest, via the one or more electrodes, with input signals (block **604).**

In embodiments, may comprise receiving response signals as a result of subjecting the patient site of interest with input signals (block **606).**

In embodiments, the method may further comprise determining, based on the received response signals, an output pertaining to a physiological phenomenon with respect to the patient site of interest (block **608).**

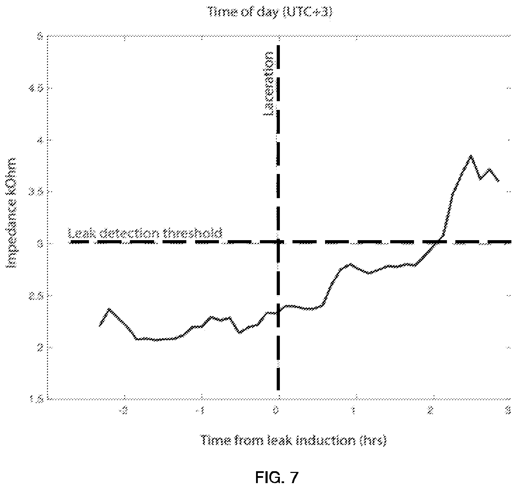

Referring now to **FIG. 7****,** the solid line shows impedance as a function of time. The horizontal dashed line indicates a leak detection threshold and the vertical dashed line indicates a time of laceration. When measured impedance crosses the threshold for a certain period of time (e.g., > 10min), an output indicative of anastomotic leak is provided. The threshold can for example be fixed, predetermined, or can change in a linear or a non-linear manner.

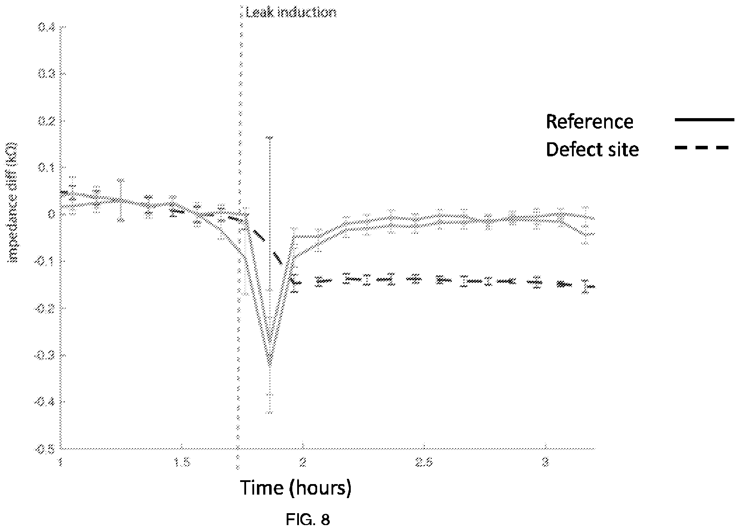

Further reference is made to **FIG. 8** in which the dashed line indicates the impedance difference from baseline measured 10mm from the leak site prior and after the induction of the defect. The sold line indicates impedance of two reference electrodes recorded at a distance >100mm from the defect. The error bars represent standard deviation of impedance measured over 2 minutes per sample. Overall separation 10 minutes after the induction of the defect reflects p<0.001

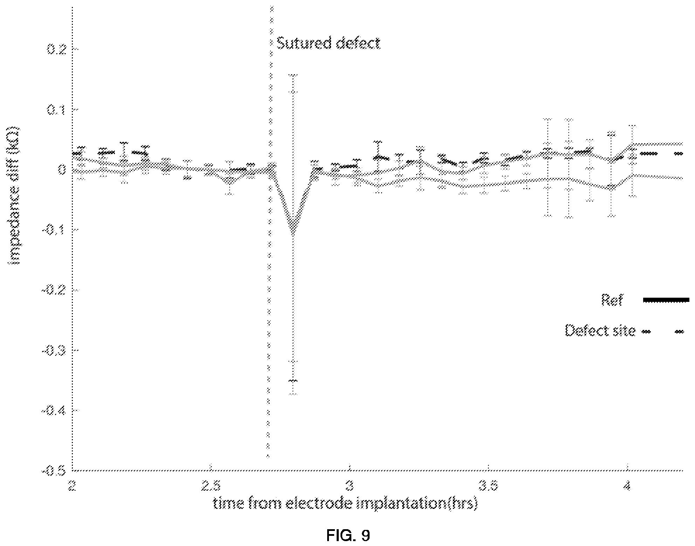

**FIG. 9** shows impedance measurement in an animal with a sutured defect (no leak). In this case there is no separation over time between the recorded impedance before and after the induction of the leak.

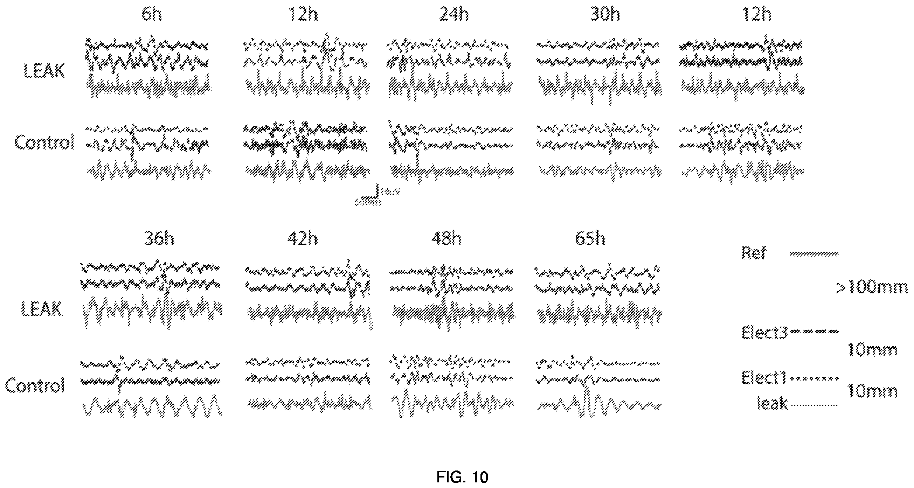

**FIG. 10** show representative electrophysiological signals from electrodes implanted 10mm, 20mm and >100mm from the defect in a chronic leak animal model and control animal model with healing defect. It is noted that there is typically better correlation between the reference and defect signals recorded in the control animal compared to the leak animal model.

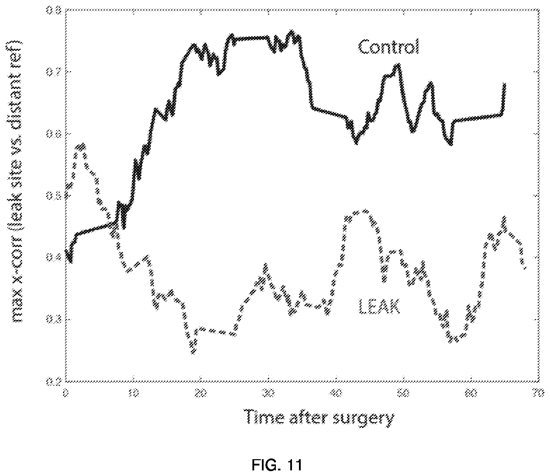

**FIG. 11** shows maximum cross correlation value ("correlation coeff") of leak animal model vs. control animal model over time. The leak model shows declining correlation between the healthy reference site and the damaged site while the correlation improves on the control model, in line with the physiological condition in the respective animals.

The various features and steps discussed above, as well as other known equivalents for each such feature or step, can be mixed and matched by one of ordinary skill in this art to perform methods in accordance with principles described herein. Although the disclosure has been provided in the context of certain embodiments and examples, it will be understood by those skilled in the art that the disclosure extends beyond the specifically described embodiments to other alternative embodiments and/or uses and obvious modifications and equivalents thereof. Accordingly, the disclosure is not intended to be limited by the specific disclosures of embodiments herein.

Any digital computer system, module and/or engine exemplified herein can be configured or otherwise programmed to implement a method disclosed herein, and to the extent that the system, module and/or engine is configured to implement such a method, it is within the scope and spirit of the disclosure. Once the system, module and/or engine are programmed to perform particular functions pursuant to computer readable and executable instructions from program software that implements a method disclosed herein, it in effect becomes a special purpose computer particular to embodiments of the method disclosed herein. The methods and/or processes disclosed herein may be implemented as a computer program product that may be tangibly embodied in an information carrier including, for example, in a non-transitory tangible computer-readable and/or non-transitory tangible machine-readable storage device. The computer program product may directly loadable into an internal memory of a digital computer, comprising software code portions for performing the methods and/or processes as disclosed herein. The term "non-transitory" is used to exclude transitory, propagating signals, but to otherwise include any volatile or non-volatile computer memory technology suitable to the application.

Additionally or alternatively, the methods and/or processes disclosed herein may be implemented as a computer program that may be intangibly embodied by a computer readable signal medium. A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a non-transitory computer or machine-readable storage device and that can communicate, propagate, or transport a program for use by or in connection with apparatuses, systems, platforms, methods, operations and/or processes discussed herein.

The terms "non-transitory computer-readable storage device" and "non-transitory machine-readable storage device" encompasses distribution media, intermediate storage media, execution memory of a computer, and any other medium or device capable of storing for later reading by a computer program implementing embodiments of a method disclosed herein. A computer program product can be deployed to be executed on one computer or on multiple computers at one site or distributed across multiple sites and interconnected by one or more communication networks.

These computer readable and executable instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable and executable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable and executable instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

In the discussion, unless otherwise stated, adjectives such as "substantially" and "about" that modify a condition or relationship characteristic of a feature or features of an embodiment of the invention, are to be understood to mean that the condition or characteristic is defined to within tolerances that are acceptable for operation of the embodiment for an application for which it is intended.

Unless otherwise specified, the terms 'about' and/or 'close' with respect to a magnitude or a numerical value may imply to be within an inclusive range of -10% to +10% of the respective magnitude or value.

"Coupled with" means indirectly or directly "coupled with".

It should be noted that where an embodiment refers to a condition of "above a threshold", this should not be construed as excluding an embodiment referring to a condition of "equal or above a threshold". Analogously, where an embodiment refers to a condition "below a threshold", this should not to be construed as excluding an embodiment referring to a condition "equal or below a threshold". It is clear that should a condition be interpreted as being fulfilled if the value of a given parameter is above a threshold, then the same condition is considered as not being fulfilled if the value of the given parameter is equal or below the given threshold. Conversely, should a condition be interpreted as being fulfilled if the value of a given parameter is equal or above a threshold, then the same condition is considered as not being fulfilled if the value of the given parameter is below (and only below) the given threshold.

It should be understood that where the claims or specification refer to "a" or "an" element and/or feature, such reference is not to be construed as there being only one of that element. Hence, reference to "an element" or "at least one element" for instance may also encompass "one or more elements".

As used herein the term "configuring" and/or 'adapting' for an objective, or a variation thereof, implies using materials and/or components in a manner designed for and/or implemented and/or operable or operative to achieve the objective.

Unless otherwise stated or applicable, the use of the expression "and/or" between the last two members of a list of options for selection indicates that a selection of one or more of the listed options is appropriate and may be made, and may be used interchangeably with the expressions "at least one of the following", "any one of the following" or "one or more of the following", followed by a listing of the various options.

As used herein, the phrase "A,B,C, or any combination of the aforesaid" should be interpreted as meaning all of the following: (i) A or B or C or any combination of A, B, and C, (ii) at least one of A, B, and C; and (iii) A, and/or B and/or C. This concept is illustrated for three elements (i.e., A,B,C), but extends to fewer and greater numbers of elements (e.g., A, B, C, D, etc.).

As used herein, "biodegradable" materials include materials that at least partially resorb into the body or otherwise break down over time while not necessarily being absorbed within the body, and "non-biodegradable" materials include those that maintain substantial mechanical integrity over their lifetime in a body. Such "biodegradable" or "nonbiodegradable" materials are well known to those having skill in the art. In some embodiments, these materials will be biocompatible, while in other embodiments, they may be partially or fully constructed from non-biocompatible materials.

It is noted that the terms "operable to" or "operative to" can encompass the meaning of the term "adapted or configured to". In other words, a machine "operable to" or "operative to" perform a task can in some embodiments, embrace a mere capability (e.g., "adapted") to perform the function and, in some other embodiments, a machine that is actually made (e.g., "configured") to perform the function.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It should be appreciated that combination of features disclosed in different embodiments could also be included within the scope of the present invention, scope which is defined by the appended claims.

## Claims

1. A monitoring system (1000) for detecting or predicting onset of adverse physiological phenomenon in a patient, the system (1000) comprising:
at least one monitoring electrode (1110) operably coupleable with a monitoring site of interest including a surgical site, the at last one monitoring electrode having electrical characteristics that are responsive to changes in physiological characteristics of the monitoring site,
at least one reference electrode (1310) operably coupleable with a reference location for monitoring a reference site which is remote from the surgical site and where no anastomotic leakage is expected to occur; and
the at least one monitoring electrode (1110) and the at least one reference electrode (1310) each being operable to provide at least one output signal descriptive of changes in physiological characteristics of the patient;
a memory, and
a processor operable to execute instructions stored in the memory to perform:
detecting the adverse physiological phenomenon by comparing the at least one output signal produced by the at least one monitoring electrode (1110) against the at least one output signal produced by at least one reference electrode (1310);and;
**characterized in that** the at least one output signal for at least one electrode including the at least one monitoring and/or the at least one reference electrode comprises at least one impedance signal and at least one electrophysiological signal.

2. The monitoring system (1000) of claim 1 wherein the at least one output signal comprises data related to gastrointestinal (Gl) activity in the patient.

3. The monitoring system (1000) of any one or more of the preceding claims, wherein the at least one output signal is indicative of a change of an electrical property of the monitoring electrode (1110),
and the processor, in conjunction with the memory, is further configured to detect anastomotic leakage based on the change of the electrical property of the at least one monitoring electrode (1110).

4. The monitoring system (1000) of any one or more of the preceding claims, further comprising:
a first communication device (1250A) that is operably coupled with a monitoring sensor (1100) comprising the monitoring electrode (1110) employed for monitoring the patient site of interest;
a reference sensor (1300) comprising the at least one reference electrode (1310) configured to provide a reference signal;
a second communication device (1250B) that is operably coupled with the at least one reference sensor employed for monitoring the remotely located reference location,
wherein the first and the second communication devices (1250A, 1250B) are configured to perform in-body transmission of signals between the monitoring electrode (1110) and the reference electrode (1310); and
a monitor (1004) operative to communicate with the first communication device (1250A) and the second communication device (1250B).

5. The monitoring system (1000) of claim 4, wherein the at least one monitoring electrode (1110) and/or the at least one reference electrode (1310) are implantable electrodes.

6. The monitoring system (1000) of any one or more of the preceding claims, further comprising:
a fluid drainage catheter that is operably positionable to drain fluids from an internal body site of interest to outside the body of the patient,
wherein the at least one reference electrode (1310) is operably integrated in, or in fluid communication with a fluid path of the bodily fluid drainage catheter for measuring changes in the physiological characteristics of the patient.

7. The monitoring system (1000) of claim 4, further comprising:
an input device for generating and subjecting the patient site of interest with an input signal to generate the at least one output signal;
wherein the first and/or the second communication devices are operative to receive the at least one output signal for processing, by the processor, data relating to the received response signal to detect anastomotic leakage from a body organ and/or an onset of anastomotic leakage.

8. The monitoring system (1000) of claim 1, wherein the adverse-physiological phenomenon includes: tissue inflammations, tissue infections, tissue breakdown, tissue dehiscence, anastomotic leakage, or any combination of the aforesaid.

9. The monitoring system (1000) of claim 1, wherein the processor is operable to execute instructions stored in the memory to monitor a healing process associated with anastomotic leakage in the patient and/or onset of anastomotic leakage.

10. The monitoring system (1000) of claim 7, wherein the processor is operable to execute instructions stored in the memory to automatically select a characteristic of the input signal based on a reference signal provided by the at least one reference electrode.

11. The monitoring system (1000) of any one or more of the preceding claims, wherein the at least one monitoring electrode (1110) and/or the at least one reference electrode (1310) is/are operative such that a change in the measured impedance pattern, and/or in the at least one electrophysiological signal, is indicative of leakage.

12. The monitoring system (1000) of claim 1, wherein the comparison includes performing cross-correlation analysis a signal recorded by the at least one monitoring electrode at the monitoring site and a signal recorded by the at least one reference electrode.

13. The monitoring system (1000) of claim 1, wherein the at least one monitoring electrode (1110) and/or the at least one reference electrode (1310) are made of non-biodegradable, partially biodegradable or fully biodegradable material.

14. The monitoring system (1000) of claim 1, wherein electro-physiological data representing phase, and/or phase shifts over time and/or phase differences between the signals recorded from the reference site and the signals recorded from the monitoring site are also be used for detecting anastomotic leakage in the patient and/or onset of anastomotic leakage.

15. The monitoring system (1000) of claim 1, wherein the signal processing is employed for concurrent sensing or measurement of at least one impedance signal and of at least one electrophysiological signal using the same at least one monitoring and/or at least one reference electrode.

16. The monitoring system (1000) of claims 1, wherein the at least one impedance signal is in the range of 20-40 Hz and the at least one electrophysiological signal is in the range of 0-120 Hz.

17. The monitoring system (1000) of claim 7, wherein the at least one output signal is indicative of a change of an at least one electrical property probed by an at least one monitoring electrode (1110), and
wherein the processor, in conjunction with the memory, is further configured to detect anastomotic leakage based on the change of at least one electrical property probed by the at least one monitoring electrode (1110).

18. The monitoring system (1000) of claim 7, wherein the at least one impedance signal is outside the range of the at least one electrophysiological signal.

## Patentansprüche

1. Ein Überwachungssystem (1000) zum Nachweis oder zur Voraussage des Beginns eines schädlichen physiologischen Phänomens in einem Patienten, wobei das System Folgendes umfasst:
- wenigstens eine Überwachungselektrode (1110), die mit einer Überwachungsstelle von Interesse umfassend eine chirurgische Stelle operativverbindbar ist, wobei die wenigstens eine Überwachungselektrode elektrische Eigenschaften aufweist, die auf Änderungen in den physiologischen Eigenschaften der Überwachungsstelle reagieren;
- wenigstens eine Referenzelektrode (1310), die operativ mit einer Referenzposition zur Überwachung einer Referenzstelle verbindbar ist, welche von der chirurgischen Stelle entfernt ist und wo keine anastomotische Leckage zu erwarten ist; und
wobei die wenigstens eine Überwachungselektrode (1110) und die wenigstens eine Referenzelektrode (1310) jeweils im Betrieb wenigstens ein Ausgangssignal bereitstellen, das die Änderungen der physiologischen Eigenschaften des Patienten beschreibt;
- einen Speicher, und
- einen Prozessor, der im Betrieb in dem Speicher gespeicherte Instruktionen wie folgt ausführt:
o den Nachweis des schädlichen physiologischen Phänomens durch Vergleich des wenigstens einen Ausgangssignals, das von der wenigstens einen Überwachungselektrode (1100) produziert wird, mit dem wenigstens einen Ausgangssignal, das von der wenigstens einen Referenzelektrode (1310) produziert wird; und
**gekennzeichnet dadurch, dass** das wenigstens eine Ausgangssignal für wenigstens eine Elektrode umfassend die wenigstens eine Überwachungs- und/oder die wenigstens eine Referenzelektrode wenigstens ein Impedanzsignal und wenigstens ein elektrophysiologisches Signal umfasst.

2. Das Überwachungssystem (1000) von Anspruch 1, wobei das wenigstens eine Ausgangssignal Daten umfasst, die sich auf gastrointestinale (GI) Aktivität in dem Patienten bezieht.

3. Das Überwachungssystem (1000) von einem oder mehreren dervorangegangenen Ansprüche, wobei das wenigstens eine Ausgangssignal eine Änderung einer elektrischen Eigenschaft der Überwachungsselektrode (1110) anzeigt, und der Prozessor in Verbindung mit dem Speicher konfiguriert ist, eine anastomotische Leckage basierend auf der Änderung der elektrischen Eigenschaft der wenigstens einen Überwachungselektrode (1110) nachzuweisen.

4. Das Überwachungssystem (1000) von einem oder mehreren der vorangegangenen Ansprüche, zusätzlich umfassend:
- eine erste Kommunikationsvorrichtung (1250A), die operativ mit einem Überwachungssensor (1100)verbunden ist, der die Überwachungselektrode (1110) umfasst, die zur Überwachung der Stelle von Interesse des Patienten eingesetzt wird;
- einen Referenzsensor (1300) umfassend die wenigstens eine Referenzelektrode (1310), die konfiguriert ist, ein Referenzsignal bereitzustellen;
- eine zweite Kommunikationsvorrichtung (1250B), die operativ mit dem wenigstens einen Referenzsensor verbunden ist, der zur Überwachung der entfernt liegenden Referenzposition eingesetzt wird;
wobei die ersten und zweiten Kommunikationsvorrichtungen (1250A, 1250B) konfiguriert sind, eine Übertragung der Signale im Körper zwischen der Überwachungselektrode (1110) und der Referenzelektrode (1310) auszuführen; und
- einen Monitor (1004), der im Betrieb mit der ersten Kommunikationsvorrichtung (1250A) und der zweiten Kommunikationsvorrichtung (1250B) kommuniziert.

5. Das Überwachungssystem (1000) von Anspruch 4, wobei die wenigstens eine Überwachungselektrode (1110) und/oder die wenigstens eine Referenzelektrode (1310) implantierbare Elektroden sind.

6. Das Überwachungssystem (1000) von einem oder mehreren der vorangegangenen Ansprüche, zusätzlich umfassend:
- einen Flüssigkeitsdrainagekatheter, der im Betrieb positionierbar ist, um Flüssigkeiten von einer inneren Körperstelle von Interesse aus dem Körper des Patienten abzuleiten,-wobei die wenigstens eine Referenzelektrode (1310) operativ integriert ist in oder in Flüssigkeitsverbindung mit einem Flüssigkeitsweg des Flüssigkeitsdrainagekatheters im Körper steht, zur Messung von Änderungen in den physiologischen Eigenschaften des Patienten.

7. Das Überwachungssystem (1000) von Anspruch 4, zusätzlich umfassend:
ein Eingabegerät zur Generierung und Übermittlung eines Eingabesignals an die Patientenstelle von Interesse zur Generierung des wenigstens einen Ausgabesignals; wobei die ersten und/oder die zweiten Kommunikationsvorrichtungen im Betrieb das wenigstens eine Ausgabesignal zur Verarbeitung von Daten durch den Prozessor empfangen, die sich auf das empfangene Antwortsignal beziehen, um eine anastomotische Leckage von einem Organ des Körpers und/oder einen Beginn einer anastomotischen Leckage nachzuweisen.

8. Das Überwachungssystem (1000) von Anspruch 1, wobei das schädliche physiologische Phänomen die Folgenden umfasst: Gewebeentzündungen, Gewebeinfektionen, Gewebezerfall, Gewebedehiszenz, anastomotische Leckage oder eine Kombination der Vorgenannten.

9. Das Überwachungssystem (1000) von Anspruch 1, wobei der Prozessor im Betrieb Instruktionen ausführt, die im Speicher gespeichert sind, um einen Heilungsprozess zu überwachen, der mit anastomotischer Leckage in dem Patienten und/oder dem Beginn einer anastomotischen Leckage assoziiert ist.

10. Das Überwachungssystem (1000) von Anspruch 7, wobei der Prozessor im Betrieb Instruktionen ausführt, die in dem Speicher gespeichert sind, um automatisch eine Eigenschaft des Eingangssignals auf der Basis eines Referenzsignals auszuwählen, das durch die wenigstens eine Referenzelektrode bereitgestellt wird.

11. Das Überwachungssystem (1000) von einem oder mehreren der vorangegangenen Ansprüche, wobei die wenigstens eine Überwachungselektrode (1110) und/oder die wenigstens eine Referenzelektrode (1310) im Betrieb feststellen, dass eine Änderung in dem gemessenen Impedanzmuster, und/oder in dem wenigstens einen elektrophysiologischen Signal eine Leckage anzeigt.

12. Das Überwachungssystem (1000) von Anspruch 1, wobei der Vergleich die Durchführung einer Kreuz-Korrelations-Analyse eines Signals umfasst, das durch die wenigstens eine Überwachungselektrode an der Überwachungsstelle aufgenommen wurde, und eines Signals, das durch die wenigstens eine Referenzelektrode aufgenommen wurde.

13. Das Überwachungssystem (1000) von Anspruch 1, wobei die wenigstens eine Überwachungselektrode (1110) und/oder die wenigstens eine Referenzelektrode (1310) aus nicht biologisch abbaubarem, teilweise biologisch abbaubarem oder vollständig biologisch abbaubarem Material hergestellt sind.

14. Das Überwachungssystem (1000) von Anspruch 1, wobei elektrophysiologische Daten, die eine Phase und/oder Phasenverschiebungen mit der Zeit und/oder Phasenunterschiede zwischen den Signalen darstellen, die von der Referenzstelle und den Signalen, die von der Überwachungsstelle aufgenommen wurden, auch zum Nachweis einer anastomotischen Leckage in dem Patienten und/oder des Beginns eine anastomotischen Leckage verwendet werden.

15. Das Überwachungssystem (1000) von Anspruch 1, wobei die Signalverarbeitung zum gleichzeitigen Abtasten oder Messen wenigstens eines Impedanzsignals und wenigstens eines elektrophysiologischen Signals unter Verwendung der gleichen wenigstens einen Überwachungs- und/oder der wenigstens einen Referenzelektrode eingesetzt wird.

16. Das Überwachungssystem (1000) von Anspruch 1, wobei das wenigstens eine Impedanzsignal in dem Bereich von 20 bis 40 Hz liegt und das wenigstens eine elektrophysiologische Signal im Bereich von 0 bis 120 Hz liegt.

17. Das Überwachungssystem (1000) von Anspruch 7, wobei das wenigstens eine Ausgangssignal eine Änderung wenigstens einer elektrischen Eigenschaft anzeigt, die mittels der wenigstens einen Überwachungselektrode (1110) gemessen wurde, und wobei der Prozessor in Verbindung mit dem Speicher zudem konfiguriert ist, eine anastomotische Leckage basierend auf der Änderung wenigstens einer elektrischen Eigenschaft zu bestimmen, die mit der wenigstens einen Überwachungselektrode (1100) gemessen wurde.

18. Das Überwachungssystem (1000) von Anspruch 7, wobei das wenigstens eine Impedanzsignal ausserhalb des Bereichs des wenigstens einen elektrophysiologischen Signals liegt.

## Revendications

1. Système de surveillance (1000) permettant de détecter ou de prédire l'apparition d'un phénomène physiologique défavorable sur un patient, le système (1000) comprenant :
au moins une électrode de surveillance (1110) pouvant être couplée de manière exploitable à un site d'intérêt de surveillance incluant un site chirurgical, l'au moins une électrode de surveillance présentant des caractéristiques électriques qui sont sensibles à des changements de caractéristiques physiologiques du site de surveillance,
au moins une électrode de référence (1310) pouvant être couplée de manière exploitable à un emplacement de référence pour surveiller un site de référence qui est distant du site chirurgical et où aucune fuite anastomotique ne devrait se produire ; et
l'au moins une électrode de surveillance (1110) et l'au moins une électrode de référence (1310) pouvant chacune être exploitées pour fournir au moins un signal de sortie descriptif de changements de caractéristiques physiologiques du patient ;
une mémoire, et
un processeur pouvant être exploité pour exécuter des instructions stockées dans la mémoire pour :
détecter le phénomène physiologique défavorable en comparant l'au moins un signal de sortie produit par l'au moins une électrode de surveillance (1110) à l'au moins un signal de sortie produit par au moins une électrode de référence (1310) ; et
**caractérisé en ce que** l'au moins un signal de sortie pour au moins une électrode incluant l'au moins une électrode de surveillance et/ou l'au moins une électrode de référence comprend au moins un signal d'impédance et au moins un signal électrophysiologique.

2. Système de surveillance (1000) selon la revendication 1, dans lequel l'au moins un signal de sortie comprend des données liées à l'activité gastro-intestinale (GI) du patient.

3. Système de surveillance (1000) selon l'une quelconque des revendications précédentes, dans lequel l'au moins un signal de sortie est indicatif d'un changement d'une propriété électrique de l'électrode de surveillance (1110),
et le processeur, en combinaison avec la mémoire, est en outre configuré pour détecter une fuite anastomotique sur la base du changement de la propriété électrique de l'au moins une électrode de surveillance (1110).

4. Système de surveillance (1000) selon l'une quelconque des revendications précédentes, comprenant en outre :
un premier dispositif de communication (1250A) qui est couplé de manière exploitable à un capteur de surveillance (1100) comprenant l'électrode de surveillance (1110) employée pour surveiller le site d'intérêt du patient ;
un capteur de référence (1300) comprenant l'au moins une électrode de référence (1310) configurée pour fournir un signal de référence ;
un deuxième dispositif de communication (1250B) qui est couplé de manière exploitable à l'au moins un capteur de référence employé pour surveiller l'emplacement de référence situé à distance,
dans lequel le premier et le deuxième dispositif de communication (1250A, 1250B) sont configurés pour réaliser une transmission de signaux interne au corps entre l'électrode de surveillance (1110) et l'électrode de référence (1310) ; et
un moniteur (1004) opérationnel pour communiquer avec le premier dispositif de communication (1250A) et le deuxième dispositif de communication (1250B).

5. Système de surveillance (1000) selon la revendication 4, dans lequel l'au moins une électrode de surveillance (1110) et/ou l'au moins une électrode de référence (1310) sont des électrodes implantables.

6. Système de surveillance (1000) selon l'une quelconque des revendications précédentes, comprenant en outre :
un cathéter de drainage de fluide qui peut être positionné de manière exploitable pour drainer des fluides d'un site d'intérêt corporel interne vers l'extérieur du corps du patient,
dans lequel l'au moins une électrode de référence (1310) est intégrée de manière opérationnelle dans, ou en communication fluidique avec un chemin de fluide du cathéter de drainage de fluide corporel pour mesurer des changements des caractéristiques physiologiques du patient.

7. Système de surveillance (1000) selon la revendication 4, comprenant en outre :
un dispositif d'entrée permettant de générer et de soumettre le site d'intérêt du patient à un signal d'entrée pour générer l'au moins un signal de sortie ;
dans lequel le premier et/ou le deuxième dispositif de communication sont opérationnels pour recevoir l'au moins un signal de sortie pour le traitement, par le processeur, de données relatives au signal de réponse reçu pour détecter la fuite anastomotique d'un organe corporel et/ou une apparition d'une fuite anastomotique.

8. Système de surveillance (1000) selon la revendication 1, dans lequel le phénomène physiologique défavorable inclut : des inflammations des tissus, des infections des tissus, des ruptures de tissus, des déhiscences des tissus, une fuite anastomotique ou une combinaison quelconque des événements ci-dessus.

9. Système de surveillance (1000) selon la revendication 1, dans lequel le processeur est exploitable pour exécuter des instructions stockées dans la mémoire pour surveiller un processus de cicatrisation associé à la fuite anastomotique du patient et/ou à l'apparition d'une fuite anastomotique.

10. Système de surveillance (1000) selon la revendication 7, dans lequel le processeur est exploitable pour exécuter des instructions stockées dans la mémoire pour sélectionner automatiquement une caractéristique du signal d'entrée sur la base d'un signal de référence fourni par l'au moins une électrode de référence.

11. Système de surveillance (1000) selon l'une quelconque des revendications précédentes, dans lequel l'au moins une électrode de surveillance (1110) et/ou l'au moins une électrode de référence (1310) est/sont opérationnelle(s) de sorte qu'un changement du modèle d'impédance mesuré, et/ou dans l'au moins un signal électrophysiologique, est indicatif d'une fuite.

12. Système de surveillance (1000) selon la revendication 1, dans lequel la comparaison inclut la réalisation d'une analyse de corrélation croisée d'un signal enregistré par l'au moins une électrode de surveillance sur le site de surveillance et d'un signal enregistré par l'au moins une électrode de référence.

13. Système de surveillance (1000) selon la revendication 1, dans lequel l'au moins une électrode de surveillance (1110) et/ou l'au moins une électrode de référence (1310) sont réalisées en un matériau non-biodégradable, partiellement biodégradable ou totalement biodégradable.

14. Système de surveillance (1000) selon la revendication 1, dans lequel des données électrophysiologiques représentant une phase et/ou des changements de phase au fil du temps et/ou des différences de phase entre les signaux enregistrés depuis le site de référence et les signaux enregistrés depuis le site de surveillance doivent également être utilisées pour détecter une fuite anastomotique chez le patient et/ou l'apparition d'une fuite anastomotique.

15. Système de surveillance (1000) selon la revendication 1, dans lequel le traitement du signal est employé pour une détection ou une mesure simultanée d'au moins un signal d'impédance et d'au moins un signal électrophysiologique en utilisant la même au moins une électrode de surveillance et/ou l'au moins une électrode de référence.

16. Système de surveillance (1000) selon la revendication 1, dans lequel l'au moins un signal d'impédance est dans la plage de 20 à 40 Hz, et l'au moins un signal électrophysiologique est dans la plage de 0 à 120 Hz.

17. Système de surveillance (1000) selon la revendication 7, dans lequel l'au moins un signal de sortie est indicatif d'un changement d'une au moins une propriété électrique sondée par une au moins une électrode de surveillance (1110), et
dans lequel le processeur, en combinaison avec la mémoire, est en outre configuré pour détecter une fuite anastomotique sur la base du changement d'au moins une propriété électrique sondée par l'au moins une électrode de surveillance (1110).

18. Système de surveillance (1000) selon la revendication 7, dans lequel l'au moins un signal d'impédance est hors de la plage de l'au moins un signal électrophysiologique.
